# DEMANDE DE BREVET EUROPEEN

(11) **EP 3 689 188 A1**
(43) Date de publication de la demande: **05.08.2020**
(21) Numéro de dépôt: 20154911.0
(22) Date de dépôt: 31.01.2020
(51) Int. Cl.: A47C 19/02, A47C 21/06, A61F 5/37

(54) **MOBILIER DE COUCHAGE NOTAMMENT DESTINE A UN ENVIRONNEMENT DIFFICILE**

(30) Priorité: 01.02.2019 FR 1900993
(71) Demandeur: Mobiliss, 47110 Sainte-Livrade-sur-Lot (FR)
(72) Inventeur: GOTTI, Thierry Lucien, 47110 Sainte-Livrade-sur-Lot (FR)
(74) Mandataire: Aquinov

(57) **Abrégé**

Mobilier de couchage (1), notamment destiné à un environnement difficile, comportant un sommier qui comprend une plaque (20) et un système de fixation (6) pour sangles, caractérisé en ce que le système de fixation (6) comporte des évidements (60) traversants, en particulier de section sensiblement rectangulaire, qui sont agencés dans la plaque (20) du sommier et en sa périphérie.

## Description

L'invention concerne un mobilier de couchage destiné notamment à un environnement difficile et doté d'un système de fixation de sangles de contention.

Par l'expression, mobilier pour environnement difficile, on entend tout mobilier destiné à une chambre d'isolement prévue dans certaines institutions telles qu'une unité psychiatrique, ou environnement carcéral.

L'invention sera plus particulièrement décrite en regard d'un lit d'isolement notamment pour des unités psychiatriques, sans toutefois y être limitée.

Les chambres d'isolement ou d'apaisement sont utilisées dans des situations d'urgence pour soigner et/ou assurer la sécurité à des personnes particulièrement agitées et violentes. De fait, les mobiliers destinés à ce type de chambre sont conçus pour être confortables, durables et résistants aux tentatives de destruction tout en assurant en même temps une protection pour les personnes dangereuses pour elles-mêmes et une sécurité pour le personnel soignant.

Dans certaines situations lorsque le patient est particulièrement agité, il est nécessaire de l'immobiliser, ou une partie de son corps, sur le lit par des moyens de contention. Dans ce cas, il est prévu d'équiper le lit d'un système qui permet de fixer ces moyens de contention. Ces moyens de contention sont par exemple des sangles ou similaires qui sont disposées transversalement sur le patient alité.

Il est par exemple connu un lit possédant un sommier métallique sous forme de plaque qui est solidaire de manière indémontable et coplanaire d'un cadre tubulaire, le cadre étant doté en tant que système de fixation pour sangles, d'attaches formant des poignées en U en saillie vers le bas et dans lesquelles peuvent être passées des sangles. De plus, les pieds du lit sont formés de montants soudés au cadre et à la verticale du bord du cadre, et de traverses reliant les montants.

Cependant, la force de patients est telle que le personnel soignant peut parfois se cogner sur les poignées de fixation de sangles. De plus, un tel lit présente un piètement qui est à la verticale des bords du lit, ce qui peut gêner le personnel.

L'invention a donc pour but de proposer un mobilier de couchage qui obvie aux inconvénients précités en garantissant un mobilier solide et sécuritaire, notamment pour le patient et le personnel soignant lorsque le mobilier est utilisé en tant que lit psychiatrique.

A cet effet, l'invention concerne un mobilier de couchage, notamment destiné à un environnement difficile, comportant un sommier qui comprend une plaque (pleine) destinée à accueillir un matelas de couchage, et un système de fixation pour sangles, caractérisé en ce que le système de fixation pour sangles comporte des évidements traversants, en particulier de section sensiblement rectangulaire pour constituer des passe-sangles, qui sont agencés dans la plaque du sommier et en sa périphérie.

Ainsi, le système de fixation ne fait nullement saillie par rapport au sommier. L'intégration des passe-sangles directement dans la plaque du sommier constitue une solution au risque de blessures pouvant être engendrées sur le personnel lors d'intervention sur les patients. Cet agencement coplanaire à la plaque du sommier, sans angles, sans faire saillie, garantit de ne pas se cogner, et constitue une sécurité pour le patient et le personnel soignant.

De plus, cette intégration du système de fixation à la plaque du sommier sans être agencé à l'extérieur du sommier garantit son inaccessibilité lorsqu'un matelas de couchage est disposé sur le sommier, évitant au patient tout risque de tentation d'introduction d'un drap au travers des évidements. Pour garantir une sécurité totale, la section des évidements est avantageusement de dimension adaptée pour ne pas pouvoir passer de drap à travers.

Par ailleurs, l'intégration même du système de fixation à la plaque du sommier, procure un ensemble unitaire, plus simple de fabrication et voire plus résistant.

Plus particulièrement, le mobilier de couchage comporte un sommier qui comprend une plaque pleine et un système de fixation pour sangles (sangles de contention destinées à immobiliser une personne sur le mobilier), et un cadre qui est solidaire de la périphérie de la plaque, le mobilier étant dédié à être utilisé dans un environnement difficile, et le système de fixation pour sangles comportant des évidements traversants, en particulier de section sensiblement rectangulaire, qui sont agencés de manière discontinue dans la plaque du sommier, en sa périphérie et en limite du cadre.

Selon une caractéristique, les évidements du sommier sont usinés dans la plaque, de préférence issus de découpes sur le bord périphérique de la plaque à la manière de créneaux qui procurent une alternance de zones pleines et de décrochements. Avantageusement, le sommier comporte un cadre solidaire de la périphérie de la plaque, et les décrochements de la plaque délimitent avec le cadre lesdits évidements.

Les évidements traversants forment des fentes; ils sont donc étroits, en particulier pour éviter toute introduction d'objets plus épais que des sangles comme du drap par exemple. Les fentes sont distribuées de manière discontinue, de préférence sur toute la périphérie de la plaque, en limite du cadre.

Avantageusement, le cadre est rendu solidaire de la plaque en étant fixé à l'une des faces de la plaque, la face dite supérieure, en particulier à la périphérie de la plaque sur les zones pleines des créneaux (entre deux décrochements), en particulier par soudage lorsque le cadre et la plaque sont métalliques.

De préférence, les évidements sont répartis sur toute la périphérie du sommier, en particulier les décrochements dans la plaque sont usinés sur toute la périphérie de la plaque, permettant le passage de sangles à n'importe quel endroit.

Selon une autre caractéristique, le sommier comporte un cadre périphérique solidaire (de manière inamovible) de la plaque en étant en saillie dans une direction sensiblement perpendiculaire par rapport à l'une des faces générales de ladite plaque, en particulier par rapport à la face destinée à accueillir un matelas de couchage de sorte à constituer des moyens de calage du matelas.

Le calage du matelas dans la hauteur du cadre périphérique apporte une solution au problème des déplacements du matelas, ceci de façon transparente, c'est-à-dire sans ajouter d'éléments supplémentaires, le mobilier restant très dépouillé pour garantir la sécurité.

Le cadre périphérique est de préférence de section circulaire, évitant la présence d'arêtes ou d'angles, ce qui sécurise le mobilier vis-à-vis des risques de cognement. En variante, le cadre peut présenter une autre section tout en présentant au moins une surface bombée tournée vers l'intérieur du sommier et au droit/à la verticale des évidements, ce qui ménage (depuis le dessus du sommier) une ouverture de passage pour sangle très étroite et empêche l'introduction d'un autre objet, tout en facilitant l'introduction d'une sangle.

Avantageusement, le mobilier peut comporter une tête de lit et un pied de lit qui s'élèvent en hauteur par rapport au sommier en étant solidaires du cadre, de préférence la tête de lit et le pied de lit étant constitués de tubes, en particulier de section circulaire et avec des coudes arrondis.

Ainsi, le lit peut présenter un aspect davantage hôtelier, et la solidarisation de la tête et du pied au cadre permet de ne pas obturer les évidements de passage des sangles.

Le mobilier comporte un piètement, tel que plusieurs pieds répartis sous la surface de la plaque, le piètement étant solidaire de la face dite inférieure de la plaque et disposé à distance du bord périphérique du sommier de sorte à éviter de se cogner dans ledit piètement.

Dans la suite de la description le terme de « hauteur », les qualificatifs « supérieur », « inférieur », « haut » et « bas » d'un élément du mobilier sont utilisés dans le cadre d'une installation normale du mobilier, c'est-à-dire relatif à une notion horizontale d'agencement du sommier pour le couchage.

La présente invention est maintenant décrite à l'aide d'exemples uniquement illustratifs et nullement limitatifs de la portée de l'invention, et à partir des illustrations jointes, dans lesquelles :
[Fig. 1] représente une vue en perspective d'un exemple de mobilier doté d'un sommier selon l'invention.
[Fig. 2] représente une vue en perspective d'une variante de réalisation du mobilier de la figure 1.
[Fig. 3] représente une vue en perspective d'une autre variante de réalisation du mobilier de la figure 1.
[Fig. 4] est une vue en perspective de détail (d'un angle du sommier) et de dessus de la figure 1.
[Fig. 5] est une vue de dessous de la figure 4.
[Fig. 6] est une vue de dessus du dessous de la plaque du sommier selon l'invention, sans les moyens de piètement.

Le mobilier 1 de l'invention illustré sur les figures est plus particulièrement destiné à une utilisation dans un environnement difficile tel que dans une unité psychiatrique.

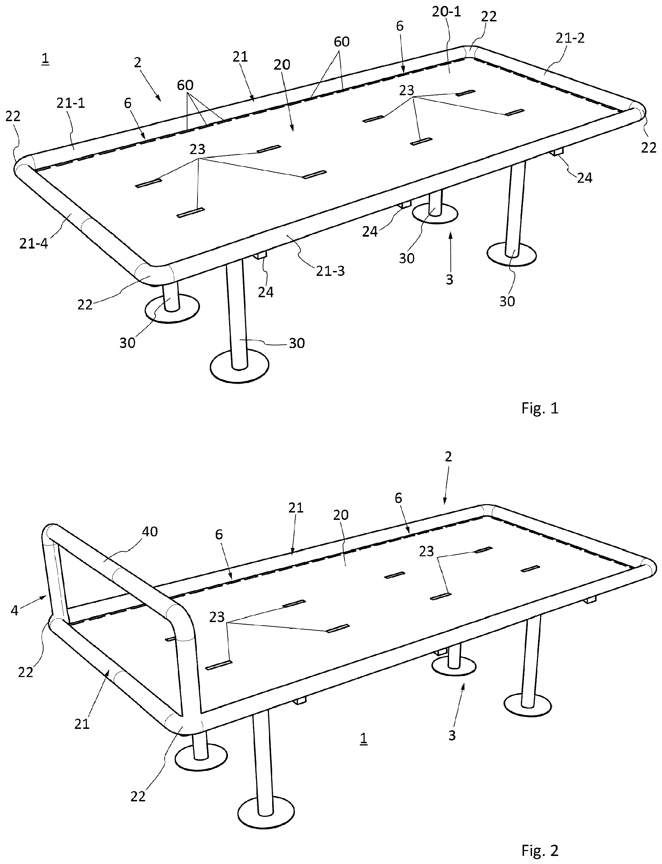

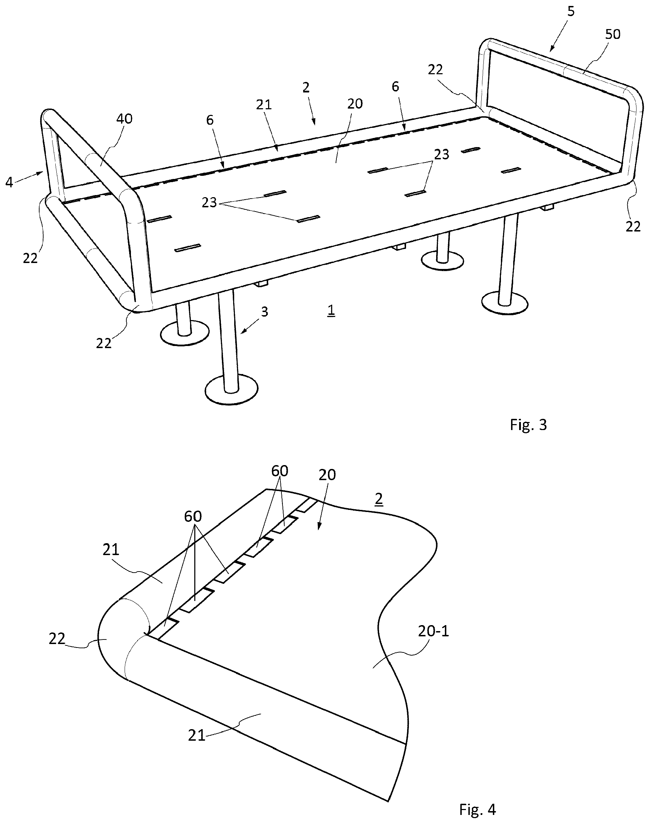

Le mobilier 1 forme un dispositif de couchage comportant au moins un sommier 2 et des moyens de piètement 3, tel que comprenant quatre pieds 30, pour agencer le sommier en hauteur. Dans l'exemple de la figure 2, le mobilier 1 comporte en outre une tête de lit 4 ; dans l'exemple de la figure 3, le mobilier 1 comporte outre la tête de lit 4, un pied de lit 5 en regard et à l'opposé de la tête de lit, donnant au mobilier un aspect de lit plutôt que de table comme dans la figure 1. En outre, le mobilier 1 comporte un système de fixation 6 de sangles de contention.

Le sommier 2 comporte d'une part une plaque pleine 20 présentant deux faces générales opposées, une face dite supérieure 20-1 et une face dite inférieure 20-2, et d'autre part, un cadre périphérique 21 qui est solidaire de la plaque 20 de manière inamovible. De préférence, l'ensemble du sommier est métallique.

Le cadre périphérique 21 est formé de profilés 21-1 à 21-4 de section circulaire qui ont été soudés entre eux par des coudes 22 à la forme arrondie. Les formes rondes participent à la sécurité du mobilier.

Selon l'invention, le cadre 21 est en saillie de la face supérieure 20-1. Le cadre 21 s'élève en hauteur vis-à-vis de la plaque 20. Ainsi, le cadre 21 constitue des moyens de butée/de calage d'un matelas destiné à être posé sur la plaque 20. A titre d'exemple préféré, pour une fabrication simple, rapide et peu coûteuse, le cadre est formé à partir de tubes métalliques de section circulaire, tels que de diamètre de l'ordre de 50 mm. Les tubes métalliques formant les côtés du cadre sont connectés par des coudes, et leur sont rendus solidaires par soudage.

Le cadre 21 est rendu solidaire de la plaque 20 comme il sera vu plus loin.

De préférence, la plaque 20 comporte des orifices traversants 23 qui sont répartis sur sa surface afin de constituer des moyens d'aération. Ces orifices sont par exemple de forme oblongue. Ils présentent des dimensions telles ne permettant pas le passage d'un drap.

En outre, des traverses inférieures 24 de renfort du sommier peuvent être prévues solidaires de la face inférieure 20-2 de la plaque 20 (longitudinalement et/ou transversalement). Elles ne dépassent pas du cadre 21. De préférence, elles se terminent au plus en limite de l'intérieur du cadre 21.

Pour les exemples de réalisation des figures 2 et 3, la tête de lit 4 et le pied de lit 5 sont de constitution similaire, faits de tubes identiques aux tubes du cadre 21 avec des coudes à 90° de forme arrondie pour relier la traverse du haut 40, respectivement 50, à deux montants espacés qui sont connectés au cadre 21. Avantageusement, les coudes 22 aux angles du cadre 21 et des têtes de lit et de pied sont des connecteurs trois axes.

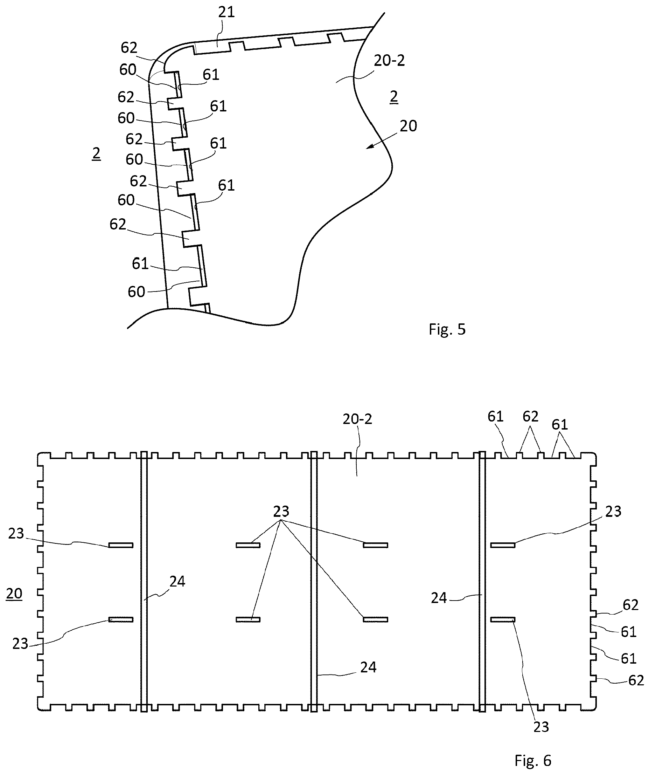

Selon l'invention, le système de fixation 6, visible plus particulièrement sur les figures 4 à 6, comportent des évidements traversants 60 qui sont usinés dans la plaque 20. De préférence, la plaque 20 présente sur son bord périphérique des découpes 61 en créneaux qui engendrent une alternance de zones pleines 62 et de décrochements en U dont la concavité du U est tournée vers l'extérieur de la plaque.

Selon l'invention, le cadre 21 est rendu solidaire, ici par soudage du fait que les éléments sont métalliques, des zones pleines 62, du côté de la face supérieure 20-1 de la plaque 20.

Les découpes/décrochements 61 avec le bord interne du cadre 21 délimitent les évidements 60.

La solidarisation du cadre 21 sur le bord des découpes 61 procurent les évidements traversants 60 de périmètre fermé en vue de dessus.

De préférence, les découpes 61 se succèdent de manière rapprochée sur l'ensemble de la périphérie de la plaque 20 de sorte à procurer des évidements 60 sur tout le tour du sommier, permettant le passage de sangles à n'importe quel endroit.

Les évidements 60 sont intégrés à la plaque 20 et sont coplanaires avec celle-ci.

Les évidements 60 présentent des dimensions adaptées pour éviter l'introduction d'un drap. A titre d'exemple préféré, la longueur des découpes 61, et donc des évidements 60 est de l'ordre de 60 mm, tandis que la longueur des zones pleines 62 est de 20 mm. On entend par « longueur » relativement aux découpes, la dimension parallèle aux côtés de la plaque. La largeur des découpes (dimension coplanaire et perpendiculaire à la longueur des découpes) est de l'ordre de 20 mm, procurant avec la tangente au cadre 21 de section circulaire, et perpendiculaire à la plaque, des évidements 60 dont la largeur d'ouverture en vue de dessus est de l'ordre de 3 mm pour que seule l'épaisseur d'une sangle de contention puisse passer. Certes, cette largeur de passage peut être considérée comme très étroite, mais contrairement à toute attente, l'utilisateur peut en réalité très aisément introduire une sangle sans manœuvre complexe, et notamment n'a pas besoin de se baisser pour visualiser par en-dessous les évidements qui sont issus des découpes 61 bien plus larges. En effet, la combinaison de la multiplicité rapprochée des évidements 60 et de la surface bombée du cadre 21 à section circulaire, permet à l'utilisateur en plaquant une sangle contre ladite surface bombée du cadre, tout en poussant vers le bas la sangle de rencontrer (quasi-)immédiatement un évidement et de passer/d'enfiler la sangle au travers sans effort de visualisation et de manipulation.

## Revendications

1. Mobilier de couchage (1), notamment destiné à un environnement difficile, comportant un sommier (2) qui comprend une plaque (20) et un système de fixation (6) pour sangles, **caractérisé en ce que** le système de fixation (6) comporte des évidements (60) traversants, en particulier de section sensiblement rectangulaire, qui sont agencés dans la plaque (20) du sommier et en sa périphérie.

2. Mobilier selon la revendication 1, **caractérisé en ce que** les évidements (60) sont usinés dans la plaque, de préférence sont issus de découpes sur le bord périphérique de la plaque à la manière de créneaux qui procurent une alternance de zones pleines (62) et de décrochements (61).

3. Mobilier selon la revendication précédente, **caractérisé en ce que** le sommier comporte un cadre (31) solidaire de la périphérie de la plaque (20), et **en ce que** les décrochements (61) de la plaque délimitent avec le cadre (21) lesdits évidements (60).

4. Mobilier selon les revendications 2 et 3, **caractérisé en ce que** le cadre (21) est rendu solidaire de la plaque (20) au niveau des zones pleines (62) des créneaux, en particulier par soudage lorsque le cadre et la plaque sont métalliques.

5. Mobilier selon l'une quelconque des revendications précédentes, **caractérisé en ce que** les évidements (60) sont répartis sur toute la périphérie du sommier.

6. Mobilier selon l'une quelconque des revendications précédentes, **caractérisé en ce que** le sommier (2) comporte un cadre périphérique (21) qui est solidaire de la plaque (20) en étant en saillie dans une direction sensiblement perpendiculaire par rapport à l'une des faces générales de ladite plaque, en particulier par rapport à la face dite supérieure (20-1) destinée à accueillir un matelas de couchage de sorte à constituer des moyens de calage du matelas.

7. Mobilier selon l'une quelconque des revendications précédentes, **caractérisé en ce que** le sommier (2) est métallique et comporte un cadre (21) périphérique qui est solidaire de la plaque par soudage, de préférence le cadre étant tubulaire, en particulier de section circulaire.

8. Mobilier selon la revendication 6 ou 7, **caractérisé en ce que** le cadre (21) périphérique est de section circulaire, ou présente au moins une surface bombée tournée vers l'intérieur du sommier (2) et au droit des évidements (60).

9. Mobilier selon l'une quelconque des revendications précédentes, **caractérisé en ce qu'**il comporte une tête de lit (4) et un pied de lit (5) qui s'élèvent en hauteur par rapport au sommier (2) en étant solidaires du cadre, de préférence la tête de lit et le pied de lit étant constitués de tubes, en particulier de section circulaire et avec des coudes arrondis.

10. Mobilier selon l'une quelconque des revendications précédentes, **caractérisé en ce qu'**il comporte des moyens de piètement (3), tel que plusieurs pieds (30) répartis sous la surface de la plaque (20), le piètrement étant solidaire de la face dite inférieure (20-2) de la plaque (20) et disposé à distance du bord périphérique du sommier (2).
